# FASCICULE DE BREVET EUROPEEN

(11) **EP 0 332 501 B1**
(45) Date de publication et mention de la délivrance du brevet: **11.05.1994**
(21) Numéro de dépôt: 89400555.2
(22) Date de dépôt: 28.02.1989
(51) Int. Cl.: B01F 17/00, A61K 7/00

(54) **Utilisation de polymères de type polyacrylate en tant qu'agents stabilisants de dispersions constitués d'une phase insoluble dans l'eau dans une phase aqueuse, dispersions obtenues et leur préparation**
Verwendung von Polyacrylat-Polymeren als Stabilisiermittel für Suspensionen, aus einer wasserunlösbaren Phase in einer wässerigen Phase erhaltene Suspensionen und deren Herstellung
Use of polyacrylate polymers as stabilizing agents of dispersions constituted of a water-insoluble phase in an aqueous phase, dispersions obtained, and their preparation

(30) Priorité: 01.03.1988 FR 8802559
(43) Date de publication de la demande: 13.09.1989
(73) Titulaire: LABORATOIRES Dr. N.G. PAYOT, 75001 Paris (FR)
(72) Inventeur: Fructus, Alain, F-92400 Courbevoie (FR)
(74) Mandataire: Boulinguiez, Didier

(56) Documents cités:
- EP-A- 0 121 874
- EP-A- 0 151 984
- EP-A- 0 194 621
- DE-C- 1 211 655
- FR-A- 2 026 790
- FR-A- 2 187 826
- FR-A- 2 240 755
- FR-A- 2 324 352
- US-A- 2 308 236
- US-A- 3 996 309

## Description

La présente invention concerne le domaine des dispersions aqueuses d'une phase insoluble dans l'eau, lesdites dispersions étant exemptes d'agent émulsifiant. Elle a plus particulièrement pour objet l'utilisation de polymères particuliers de type polyacrylate en tant qu'agents stabilisants pour de telles compositions.

Dans le domaine cosmétique, de très nombreuses compositions, crèmes, laits, lotions, maquillages sont constituées par une émulsion, c'est-à-dire par une dispersion plus ou moins fine d'une phase dans une autre phase. Les deux phases sont insolubles l'une dans l'autre. Il y a en général une phase dite aqueuse dont le constituant majeur est l'eau, et une phase dite grasse qui est un mélange plus ou moins complexe de constituants huileux ou cireux tous insolubles dans l'eau. L'émulsion peut être eau-dans-huile, c'est-à-dire que la phase dispersée est la phase aqueuse, ou huile-dans-eau qui est l'inverse.

Dans d'autres domaines, notamment dans le domaine alimentaire ou pharmaceutique, on utilise également des compositions d'ingrédients actifs qui se présentent sous la forme de dispersions d'une phase insoluble dans l'eau dans une phase aqueuse. Ces compositions sont notamment les compositions de protéines, de pigments, d'agents conservateurs ou de médicaments.

Quel que soit le domaine considéré, quand on disperse une phase dans une autre, la tendance des gouttelettes de la phase dispersée est de coalescer pour former des gouttes de plus en plus grosses, pour finir en une phase continue, comme si la phase n'avait pas été dispersée. Il y a alors séparation des phases suivant la densité des deux phases.

Afin de stabiliser les gouttelettes et donc d'empêcher une émulsion de se "casser", on emploie classiquement des agents émulsifiants. Par "agents émulsifiants", on désigne dans la présente description des molécules comportant une partie lipophile et une partie hydrophile. Lorsqu'elles sont utilisées par exemple au sein d'une dispersion huile-dans-eau, ces molécules se disposent à l'interface des gouttelettes d'huile, la partie hydrophile de ces molécules étant dans la phase aqueuse externe de cette dispersion. Cette disposition de la partie hydrophile crée autour des gouttelettes une protection contre la coalescence des gouttelettes entre elles et stabilise donc l'émulsion.

Il existe une grande variété d'agents émulsifiants qui permettent de réaliser toutes sortes d'émulsions. Bien que sélectionnés pour leur innocuité vis-à-vis de la peau, certains émulsifiants présentent des inconvénients dans les domaines cosmétique ou alimentaire. Il ne sont, par exemple en cosmétique, pas tous bien tolérés par tous les types de peau. Ils peuvent également favoriser la pénétration de produits contenus dans l'émulsion à un niveau de la peau non désiré. Ils peuvent également inhiber l'activité de certains principes actifs ou de certains conservateurs. Enfin, ils permettent à de l'eau extérieure (pluie, toilette, bains de mer) de remettre en émulsion les phases grasses déposées sur la peau dans le but de la protéger de la déshydratation.

Certains agents émulsifiants peuvent également se substituer aux phospholipides de la peau formant les bicouches membranaires. Ils peuvent alors désorganiser les parois des cellules et/ou extraire de ces parois des protéines membranaires dont la présence est indispensable à le vie des cellules.

L'utilisation d'émulsifiants présente également des inconvénients dans le domaine alimentaire.

Etant donné les inconvénients ci-dessus, il est hautement souhaitable de disposer de dispersions stables, exemptes d agent émulsifiant.

On a maintenant trouvé que les dispersions d'une phase insoluble dans l'eau dans une phase aqueuse pouvaient être stabilisées par un polymère de type polyacrylate.

L'utilisation de polymères pour stabiliser une émulsion à côté des émulsifiants conventionnels est classique. Toutefois, la stabilisation de dispersions avec seulement des polymères requiert soit l'utilisation de polymères particuliers, tels que par exemple ceux commercialisés sous la dénomination CARBOPOL® 1342, soit l'utilisation d'un fort pourcentage de polymères qui rend la dispersion inutilisable, notamment en cosmétique.

On a trouvé que les polymères de la famille de polyacrylates définis ci-après pouvaient être utilisés comme agents stabilisants de dispersions constituées d'une phase insoluble dans l'eau dans une phase aqueuse.

Ainsi, la présente invention a pour objet l'utilisation, en tant qu'agent stabilisant de dispersions constituées d'une phase insoluble dans l'eau dans une phase aqueuse, d'un polymère contenant des unités de formule : 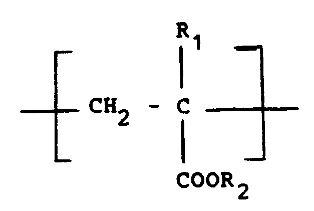
dans laquelle :
R₁ est l'hydrogène ou un radical alkyle ayant de 1 à 10 atomes de carbone;
R₂ est un reste hydrocarboné linéaire ou ramifié, contenant 1 à 12 atomes de carbone, substitué par un ou plusieurs groupes hydroxy éventuellement estérifiés.

A titre d'exemples de radical R₂, on peut citer les restes de glycérol, de propylèneglycol, de butylèneglycol, d'hexylèneglycol et les restes d'esters de glycol.

L'invention a également pour objet les dispersions constituées d'une phase insoluble dans l'eau dans une phase aqueuse, stabilisées avec l'agent stabilisant selon l' invention et plus particulièrement les compositions cosmétiques, alimentaires ou pharmaceutiques constituées par de telles dispersions.

Les polymères de type polyacrylate utilisés comme stabilisants selon l'invention peuvent être obtenus par réaction d'un alcoolate alcalin avec un polymère d'acide acrylique convenablement sustitué selon des procédés classiques, bien connus de l'homme du métier.

La viscosité Brookfield des polymères mis en oeuvre selon l'invention, mesurée à 20°C, est comprise entre 200.000 et 800.000 mPa.s, et de préférence entre 400.000 et 700.000 mPa.s.

Par exemple, l'un des polymères de cette famille, à savoir le polyglycérylméthacrylate répondant à la formule I ci-dessus dans laquelle R₁ est un groupe méthyle, R₂ est un reste de glycérol et dont la viscosité Brookfield mesurée à 20°C est d'environ 500.000 mPa.s., est connu sous la dénomination commerciale LUBRAJEL® et se présente sous la forme d'un gel aqueux contenant un peu de propylèneglycol.

L'agent stabilisant selon l'invention est utilisé à raison de 1 à 40%, de préférence de 10 à 25% en poids par rapport au poids total de la dispersion d'une phase insoluble dans l'eau dans une phase aqueuse. Selon un mode de réalisation préféré, on peut utiliser le stabilisant selon l'invention en combinaison avec un agent gélifiant couramment utilisé dans les domaines cosmétique, alimentaire ou pharmaceutique.

Des exemples de gélifiants appropriés dans le domaine cosmétique sont :
1 - les gélifiants d'origine naturelle, tels que les carraghénanes purs (formes Kappa, Lambda, Iota, Mu, Nu), la gomme de karaya, la gomme adragante, la gomme guar, la farine de caroube, le mélange de farine de caroube et d'extraits d'algues rouges, les alginates, les pectines, la gomme arabique et l'agar-agar;
2 - les gélifiants issus de biotechnologies, tels que la gomme xanthane (Rhodopol®) et les scléroglycannes (Amigel®);
3 - les dérivés de cellulose, tels que les carboxyméthylcelluloses, notamment leurs sels de sodium (Blanose®), les hydroxyéthylcelluloses (Cellobond®) et les hydroxypropylcelluloses (Klucel®);
4 - les polymères carboxyvinyliques, tels que les produits connus sous la dénomination commerciale Carbopol®;
5 - les dérivés de guar, tels que l'hydroxypropyl guar connu sous la dénomination commerciale Jaguar®;
6 - le polymère d'oxyde d'éthylène connu sous la dénomination commerciale Polyox®.

La phase insoluble des compositions selon l'invention peut être constituée d'un ou plusieurs produits insolubles dans l'eau, tels que les corps gras, les protéines, les pigments, les agents conservateurs ou les principes actifs utilisés en cosmétique ou en pharmacie.

Des exemples de tels produits, qui peuvent donc être utilisés seuls ou en mélange, sous réserve de leur compatibilité sont:
1 - les hydrocarbures paraffiniques, isoparaffiniques ou naphténiques comme : la vaseline, l'huile de vaseline, le squalène, le perhydrosqualène, le polyisobutène, le dioctylcyclohexane, la paraffine;
2 - les esters d'acides organiques saturés ou insaturés, linéaires ou ramifiés, et d'alcools, monoalcools ou polyols comme les triglycérides : huile de tournesol, de ricin, d'avocat, de son, de riz, de bourrache, de carthame, de babassu, de macadamia, d'onagre, de rosier muscat, de camélia, de bombyx mori, de sésame, de carotte, de thé, de pin parasol, de noisettes, de germe de blé, d'amandes douces, de sisymbrium, d'olives, de hoplesethys atlanticas (cropure or);
   les esters d'acides organiques de propylèneglycol (dicaprate/dicaprylate de propylèneglycol, dioctanoate de propylèneglycol, myristate de propylèneglycol);
   les esters d'acides organiques, saturés ou insaturés, linéaires ou ramifiés, de monoalcools linéaires ou ramifiés, saturés ou insaturés, tels que l'arachidylbébénate, l'arachidyl-propionate, l'octyldodécyl-myristate, le bénényl-bébénate, le myristyl-myristate, le myristyl-lactate, l'isocétyl-stéarate, l'hydroxy-stéarate d'éthyl-2-hexyle, le linoléate d'éthyle, l'oléyl-éruccite, le palmitate d'éthyl-2-hexyle, le palmitate de cétyle, l'isostéarate d'isopropyle, le ricinoléate de cétyle, l'huile de jojoba, le 9,10-dihydroxystéarate de méthyle, l'hydroxyoctacosanyl-hydroxystéarate et le myristate d'isotridécyle;
3 - les alcools organiques, saturés ou insaturés, linéaires ou ramifiés, comme l'alcool cétylique, l'alcool oléique;
4 - les acides organiques, saturés ou insaturés, linéaires ou ramifiés, comme l'acide stéarique, l'acide ricinoléique, l'acide isostéarique, l'acide oléique, l'acide 12-hydroxystéarique;
5 - les esters de diacides organiques et d'alcools organiques saturés ou insaturés, linéaires ou ramifiés, tels que le dioctyladipate, le di-éthyl-2-hexyl-maléate, le di-isopropyladipate, le dioctylsuccinate, le di-isostéaryl-maléate, l'adipate de cétyle;
6 - les esters de l'acide néopentanoïque, tels que l'isostéaryl-néopentanoate, le néopentylglycol-dioctanoate, les esters de l'acide pelargonique (dipélargonate de propylèneglycol), et les esters ci-après : propionate de myristyle propoxylé, octyldécyl-stéaroyl-stéarate, glycéryl-triacétyl-ricinoléate, di-isopropyl-dimérate, tri-isostéaryl-trimérate, dipentaérythritol-12-hydroxystéarate, tétracaprate/caprylate de pentaérythritol;
7 - les silicones, linéaires, cycliques, greffés, tels que les produits ci-après : polydiméthyl-cyclosiloxane ("DC 344, DC 345"), diméthyl-siloxane (ABIL 100® ou ABIL® K4), polyphénylméthyl-siloxane ("DC 556"), triméthyl-stéaroxysilane/alcool cétylique ("DCQ 5 0158 A"), diméthyl-triméthyl-polysiloxane ("DC QF 1 - 3593 A");
8 - les cires d'origine végétale ou animale (mélanges très complexes) ou leurs substituts de synthèse, tels que les cires d'abeilles, de carnauba, de candellila, le beurre de karité, la cire de riz, la cire essentielle de cassie, la cire essentielle de narcisse, la cire essentielle de rose, la lanoline et ses dérivés : huile de lanoline, cire de lanoline, les alcools de lanoline, les acides de lanoline, le lanolate d'isopropyle, la lanoline hydroxylée;
9 - les filtres solaires liposolubles, comme le 4-t-butyl-4′-méthoxy-dibenzoylméthane (Parsol® 1789), la benzylidène-3-bornanone-2, le méthoxy-4-cinnamate de cyclohexyle, l'acide glycéryl-para-amino-benzoïque, l'acide octyldiméthyl-para-amino-benzoïque, l'acide 4-amino-benzoïque, l'homosalate, l'oxybenzone, le 4-méthoxy-cinnamate d'éthyl-2-hexyle et le salicylate d'éthyl-2-hexyle;
10- les repousseurs d'insectes, comme l'acétyl-3-éthylaminopropionate de N-butyle ou le diéthylamide-N,N-caprylique;
11- les vitamines, comme les tocophérols, l'acétate de tocophérol, le palmitate de la vitamine A, le d-panthénol, le d-panthényltriacétate, le cis-cis-linoléate d'éthyle;
12- les agents conservateurs, comme le phénoxyéthanol, l'alcool dichloro-benzylique, le biosol, le paraoxybenzoate de méthyle, le paraoxybenzoate de propyle, le paraoxybenzoate d'éthyle;
13- les principes acitfs insaponifiables de luzerne, d'avocat, de soja, les mélanges de tocophérols naturels, l'α-bisabolol, le palmitate de vitamine A, le γ-orizanol, les protéines, les enzymes, les peptides, les polypeptides, les fragments de d'ADN et de d'ARN;
14- les stérols, tels que le cholestérol, le β-sistostérol, le stigmastérol et le campestérol;
15- les pigments (traités ou non en surface par des amino-acides, des savons métalliques, des silicones, de la lécithine), tels que le dioxyde de titane (rutile, anatase), l'oxyde de fer, le talc, la sérécite, le mica, le mica/titane, le kaolin, l'oxychlorure de bismuth, l'ultra-marine rouge, violet, bleu, rose, vert, la poudre d'aluminium, l'oxyde de chrome, l'hydroxyde de chrome, la poudre de cuivre ou de bronze, le ferrocyanure ferrique, l'oxyde de zinc, le carmin, la laque d'aluminium des colorants CI ou colorants CI couramment utilisés dans le domaine cosmétique;
16- les charges diverses, telles que l'amidon de riz, l'amidon de riz modifié, la lauryl-lysine, la poudre de soie, le polydivinylbenzène, l'amidon de maïs, l'amidon de maïs modifié;
17- les agents anti-oxydants,tels que par exemple le butyl-hydroxyanisole, le butyl-hydroxytoluène, le dodécylgallate, le propylgallate, l'o-toluyldiguamide.

La phase aqueuse des dispersions stabilisées selon l'invention est constituée par l'eau ou des solutions aqueuses couramment utilisées pour la fabrication de telles dispersions, telles que les solutions hydroalcooliques.

Cette phase aqueuse peut également contenir une ou plusieus substances hydrosolules, notamment des substances cosmétiques hydrosolubles. Des exemples de telles substances cosmétiques hydrosolubles sont :
- les agents hydratants, tels que l'acide pyrrolidonecarboxylique et ses sels, les facteurs hydratants naturels (N.M.F.) reconstitués, l'acide lactique et ses sels, des mélanges d'oses et polyoses comme la pentavitine, les acides aminés, les peptides, les polypeptides;
- les agents de brunissage artificiel, tels que la déshydroxyacétone;
- les filtres solaires hydrosolubles;
- les agents régulateurs de la transpiration, antiperspirants, déodorants;
- les extraits végétaux;
- les extraits biologiques animaux ou végétaux, tels que protéines, peptides, polypeptides, partiellement hydrolysés ou non, le liquide amniotique, les facteurs de croissance, les enzymes, les extraits de foie, de rate, de thymus, de paroi duodénale, les dialysats de sang de veau, l'acide hyaluronique, les glycoprotéines, les protéines globulaires, les mucopolysaccharides;
- les produits obtenus par biotechnologie (filtrats cellulaires, peptides, facteurs de respiration cellulaire);
- les sels de calcium, magnésium, sodium, potassium;
- les agents anti-séborrhéiques;
- les agents rafraîchissants;
- les agents cicatrisants;
- les produits toniques;
- les eaux parfumées;
- les agents kératolytiques, dépilatoires;
- les agents antirides;
- les facteurs de réparation des cellules trop fortement irradiées aux UV.

La présente invention concerne aussi un procédé de stabilisation d'une dispersion constituée d'une phase insoluble dans l'eau dans une phase aqueuse qui consiste à diviser, à une température comprise entre 15 et 90°C, la phase insoluble dans l'eau ou phase dispersée, dans une solution aqueuse de l'agent stabilisant et éventuellement à mélanger la phase résultante avec le complément de la phase aqueuse entre 15 et 50°C.

L'agent stabilisant peut être utilisé à l'état pur ou à l'état dilué dans l'eau, éventuellement avec un humectant, tel que ceux utilisés couramment en cosmétique.

A titre d'humectants appropriés, on pourra utiliser l'un des composés ci-après ou leurs mélanges : polyéthylèneglycol 400, propylèneglycol, glycérine, sorbitol, polypropylèneglycol 425, les éthers méthylique, éthylique, isopropylique, n-propylique, n-butylique, isobutylique, tertiobutylique, phénylique d'éthylèneglycol, de propylèneglycol, de diéthylèneglycol, de dipropylèneglycol, de tripropylèneglycol.

Grâce à l'agent stabilisant selon l'invention, on peut fabriquer des dispersions stables contenant en poids de 0,01 à 40 % de phase dispersée par rapport au poids total de la composition.

On peut ajouter à la dispersion ainsi obtenue des principes actifs solubles dans l'eau ou des suspensions aqueuses de produits insolubles ou de liposomes sans que la stabilité de la dispersion soit altérée.

Selon une variante préférée de l'invention, on peut donc ajouter à la dispersion des liposomes qui peuvent de manière classique contenir des ingrédients actifs.

L'invention va être maintenant décrite plus en détail par les exemples illustratifs ci-après.

### Exemple 1

On réchauffe à 70°C 25 g de polyméthacrylate de glycérine sous forme de gel transparent dans l'eau et le propylèneglycol, dont la viscosité brookfield mesurée à 20°C est d'environ 500.000 mPa.s. Ce polymère est connu sous le nom commercial de LUBRAJEL® MS. On y mélange 4 g de polyéthylèneglycol 400 à titre d'agent humectant.

On réchauffe par ailleurs à 70°C la phase grasse suivante :

| | |
|---|---|
| - huile d'avocat | 6,00 g |
| - huile de jojoba | 6,00 g |
| - di-iso-octylcyclohexane | 9,00 g |
| - alcool cétylique | 2,00 g |
| - cire de riz | 1,50 g |

Cette phase grasse est dispersée dans le LUBRAJEL® au moyen d'un ultra-disperseur du type ultra-turrax pendant environ 10 minutes.

La taille des gouttelettes obtenues est de l'ordre de 1 à 2 microns. On refroidit ensuite la suspension réalisée et on y ajoute 45,5 g d'eau puis 0,600 g de conservateur et 0,40 g de parfum.

La stabilité de cette pseudo-émulsion est très bonne : aucune altération après centrifugation de 15 minutes à 5000 tr/min; au moins 3 mois à la température de 42°C. Cette pseudo-émulsion peut être utilisée comme une base de jour.

### Exemple 2

On réchauffe à 70°C 20 g de LUBRAJEL® auquel on ajoute 3 g de polyéthylèneglycol 400. On ajoute le gélifiant secondaire : 0,3 g de gomme xanthane connue sous le nom commercial de RHODOPOL® 23 SC, dispersé dans 38,2 g d'eau.

La phase grasse est composée de :

| | |
|---|---|
| - huile de jojoba | 7 g |
| - huile d'avocat | 7 g |
| - alcool cétylique | 1,8 g |
| - silicone volatil (Dow Corning 344) | 1,2 g |
| - cire de riz | 0,7 g |

La phase grasse est réchauffée à 70°C puis dispersée dans la phase aqueuse préparée précédemment à l'aide d'un agitateur ordinaire et le mélange est affiné par passage dans un homogénéisateur HYDROSHEAR fabriqué par APV GAULIN International.

La taille des gouttelettes obtenues est de l'ordre de 0,8 à 2 microns. On refroidit ensuite la suspension réalisée et on ajoute 20 g d extrait de thymus sous forme de liposomes, puis 0,5 g de conservateur et 0,3 g de parfum.

La stabilité de cette pseudo-émulsion est très bonne : aucune altération après centrifugation de 15 minutes à 5000 tr/min; au moins 3 mois a la température de 42°C. Cette pseudo-émulsion peut être utilisée comme crème de nuit.

### Exemple 3

On mélange 20 g de LUBRAJEL® et 3,0 g de glycérine comme agent humectant. On ajoute 0,5 g de gélifiant secondaire : un polymère carboxyvinylique connu sous le nom de CARBOPOL® 1342, dispersé dans 34,7 g d'eau.

La phase grasse est composée de :

| | |
|---|---|
| - myristate de myristyle | 8,0 g |
| - polyisobutène | 8,0 g |
| - succinate d'éthyl-2-hexyle | 5,0 g |

La phase grasse est dispersée dans la phase aqueuse à l'aide d'un agitateur à rotor-stator, puis le mélange est affiné par deux passages dans un "Microfluidizer" fabriqué par MICROFLUIDICS CORPORATION.

La taille des gouttelettes obtenues est de l'ordre de 0,2 à 0,9 micron. On refroidit ensuite la pseudo-émulsion qui a été réchauffée par les passages au "Microfluidizer" et on ajoute 20 g de liquide amniotique sous forme de liposomes, puis 0,5 g de conservateur et 0,3 g de parfum.

La stabilité de cette pseudo-émulsion est très bonne : il n'y a pas d'altération après centrifugation de 15 minutes à 5000 tr/min. La taille des particules, la répartition des tailles varient peu dans le temps même après un séjour de plusieurs mois à la température de 42°C. Cette émulsion peut être utilisée comme un lait pour le corps.

### Exemple 4

On mélange 30 g de LUBRAJEL® et 5 g de propylèneglycol. On ajoute 1 g de gélifiant secondaire : un scléroglycanne connu sous le nom d'AMIGEL®, dispersé dans 48,2 g d'eau.

La phase grasse est composée de :

| | |
|---|---|
| - cire de candellila | 1 g |
| - huile de bourrache | 5 g |
| - beurre de karité | 5 g |
| - myristyl-éthoxypropionate | 6 g |
| - 4-méthoxy-cinnamate d'éthyl-2-hexyle | 4 g (filtre UVB) |

La phase grasse réchauffée à 75°C est dispersée dans le mélange des gels réchauffés à 75°C également, au moyen d'un agitateur à rotor-stator. Le mélange est refroidi et on y ajoute 1 g de palmitate de vitamine A, 0,5 g de conservateur et 0,3 g de parfum. Le mélange est alors affiné par passage à travers un homogénéisateur du type ALM.

La taille des gouttelettes obtenues est de l'ordre de 2 à 10 microns. La stabilité de cette pseudo-émulsion est équivalente à celle des pseudo-émulsions des exemples précédents. Le produit obtenu suivant cet exemple peut être utilisé comme crème de protection solaire.

### Exemple 5

On mélange 30 g de LUBRAJEL® et 4 g de polypropylène-glycol 425. On ajoute 1 g de gélifiant secondaire : une hydroxyéthylcellulose connue sous le nom de CELLOBOND®, dispersée dans 40 g d'eau.

La phase dispersée est composée de :

| | |
|---|---|
| - adipate de cétyle | 2 g |
| - huile de vaseline légère | 5 g |
| - huile de babassu | 5 g |
| - diméthylpolysiloxane ABIL® 100 | 2 g |
| - salicylate d'éthyl-2-hexyle | 2 g |
| - diéthylamide-N,N-caprylique | 5 g |
| - lauryl-lysine | 2 g |
| - oxyde de titane traité aux aminoacides | 1 g |
| - conservateur | 0,5 g |
| - parfum | 0,5 g |

La phase dispersée est dispersée dans le mélange des gels à l'aide d'un agitateur rotor-stator. Le mélange est ensuite affiné à l'aide d'un homogénéisateur à ultra-sons, par exemple un "VIBRA-CELL" à 20 kHz. A une certaine fréquence résonnante, il y a formation de cavitations ou bulles microscopiques qui disparaissent rapidement, engendrant des variations locales de pression jusqu'à 300 atmosphères. La taille des gouttelettes obtenues est de l'ordre de 2 à 5 microns. La stabilité de cette pseudo-émulsion est équivalente à celle obtenue dans les exemples précédents. Le produit obtenu peut être utilisé comme une crème de plein air.

### Exemple 6

On disperse 10 g de palmitate de vitamine A, 10 g de mélange de tocophérols naturels et 10 g de _{q}-orizanol dans 70 g de LUBRAJEL® à température ambiante au moyen d'un ulta-turrax pendant 3 minutes. Le mélange très stable sera ensuite facilement incorporé à une émulsion classique à basse température (30°C environ, ce qui n'altère pas ces matières actives) sans en perturber l'équilibre, et en particulier sans provoquer une augmentation notable de la taille des gouttelettes de la phase dispersée, présentant ainsi la stabilité à long terme de cette émulsion.

On peut ainsi incorporer 2 à 20 % de ce mélange dans une émulsion de type ionique ou non ionique ou mixte sans en perturber la stabilité, ni la viscosité.

### Exemple 7

On incorpore 40 g du mélange suivant dans 60 g de LUBRAJEL® à température ambiante au moyen d'un ultra-disperseur pendant 3 minutes :

| | |
|---|---|
| - phénoxyéthanol | 20 g |
| - paraoxybenzoate de méthyle | 10 g |
| - paraoxybenzoate de propyle | 5 g |
| - O-cymène-5-ol | 5 g |

Ce mélange sera ensuite incorporé facilement à une émulsion classique, à basse températue (30°C environ), pour en assurer la conservation microbiologique sans en altérer l'équilibre. En particulier, ce procédé permet de ne pas provoquer une augmentation de la taille des gouttelettes de l'émulsion (déstabilisation) avec l'addition de conservateurs.

### Exemple 8

On disperse dans 90,8 g de LUBRAJEL® le mélange suivant :

| | |
|---|---|
| - oxyde de fer jaune traité aux amino-acides | 3,0 g |
| - oxyde de fer rouge traité aux amino-acides | 0,5 g |
| - oxyde de fer noir traité aux amino-acides | 0,1 g |
| - oxyde de titane traité aux amino-acides | 1,0 g |
| - oxyde de fer jaune traité aux silicones | 3,0 g |
| - oxyde de fer rouge traité aux silicones | 0,5 g |
| - oxyde de fer noir traité aux silicones | 0,1 g |
| - oxyde de titane traité aux silicones | 1,0 g |

Cette dispersion a lieu à température ambiante pendant 3 minutes. Cette dispersion très stable sera ensuite facilement incorporée à une émulsion classique pour en faire un fond de teint. L'opération se fait à température ambiante, ce qui a l'avantage, d'une part, de préserver les traitements de surface des pigments et, d'autre part, de préserver l'équilibre de l'émulsion et en particulier la taille des gouttelettes de la phase dispersée. On évite ainsi les opérations classiques de "broyage" des pigments en phase sèche ou humide et/ou de "cuisson" des pigments dans la phase grasse.

### Exemple 9

On disperse dans 80 g de LUBRAJEL® 20 g de parfum d'une eau de toilette. La dispersion se fait à température ambiante à l'aide d'un ultra-disperseur pendant 10 minutes sous vide. Ce mélange peut ensuite être incorporé à une lotion pour le corps de la même ligne parfumée. L'utilisation de ce mélange préserve les qualités olfactives du parfum et la stabilité de l'émulsion dans laquelle on l'incorpore, alors qu'il est connu que beaucoup de parfums utilisés à plus de 2 % ont tendance à déstabiliser une émulsion.

### Exemple 10

On mélange 30 g de LUBRAJEL® et 4 g de polyéthylèneglycol 400. On ajoute 0,5 % de CARBOPOL® 1342 dispersé dans 42 g d'eau. La phase dispersée est composée de :

| | |
|---|---|
| - huile de jojoba | 3 g |
| - octyl-dodécanol | 3 g |
| - silicone volatil DC 344 | 5 g |
| - parfum | 12 g |
| - conservateur | 0,5 g |

La dispersion est effectuée dans un mélangeur à rotor-stator pendant une heure à température ambiante.

La taille des gouttelettes est de l'ordre de 2 à 5 microns. La stabilité est comparable à celle obtenue avec les pseudo-émulsions des exemples précédents.

Cette pseudo-émulsion peut être utilisée comme crème parfumée dans une ligne de parfum.

### exemple 11

On mélange 30 g de LUBRAJEL® avec 10 g d'eau.

La phase grasse est composée de :

| | |
|---|---|
| - cétéaryl octanoate | 3 g |
| - huile de ricin | 2 g |
| - triglycérides capriques/capryliques | 6 g |
| - silicone cyclique, Dow Corning 345 | 3 g |

La phase grasse est réchauffée à 70°C puis dispersée dans le mélange LUBRAJEL®/eau à l'aide d'un agitateur de type rotor/stator, de façon à obtenir une dispersion fine avec des gouttelettes de 2 à 5 microns. On refroidit ensuite la suspension et on ajoute les conservateurs [Phenonip® (conservateur à base d'esters d'acide 4-hydroxybenzoïque) : 1 g, Germall® 115 (dérivé d'hydantoïne) : 0,2 g], un parfum (0,3 g) et un agent actif (dialysat de sang de veau : 2 g) et on complète à 100 g par de l'eau.

### Exemple 12

On utilise un autre agent stabilisant selon l'invention qui est un composé de type polyacrylate de glycérol, connu sous la dénomination commerciale d'HISPAGEL® et qui se présente sous la forme d'un gel aqueux de viscosité Brookfield légèrement supérieure à 200.000 mPa.s à 20°C.

On mélange 30 g d'HISPAGEL® 100 avec 10 g d'eau et on chauffe ce mélange à 75°C.

La phase grasse est constituée de :

| | |
|---|---|
| - huile d'avocat | 6,0 g |
| - huile de jojoba | 6,0 g |
| - diisooctyl cyclohexane | 9,0 g |

La phase grasse réchauffée à 75°C est dispersée dans le mélange HISPAGEL®/eau à l'aide d'un agitateur du type rotor/stator. La suspension obtenue a des gouttelettes de taille de 1 à 5 microns.

La suspension est ensuite refroidie et on y ajoute le mélange conservateur (Unisuprol® S 25 : 0,5 g), un parfum (0,3 g) et un principe actif qui est un complexe d extrait de pollen, d'insaponifiables, d'huile de soja, d'huile d'olive et d'huile de germe de blé (Filagrinol® : 0,5 %), puis on complète à 100 g avec de l'eau.

## Revendications

1. Utilisation en tant qu'agent stabilisant de dispersions constituées d'une phase insoluble dans l'eau dans une phase aqueuse, d'un polymère de type polyacrylate constitué d'unités de formule : 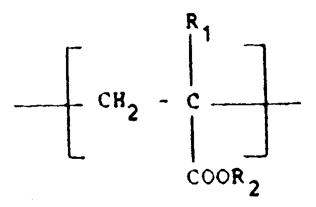 dans laquelle :
R₁ est l'hydrogène ou un radical alkyle ayant de 1 à 10 atomes de carbone ;
R₂ est un reste hydrocarboné, linéaire ou ramifié, contenant 1 à 12 atomes de carbone, substitué par un ou plusieurs groupes hydroxy éventuellement estérifiés ; la viscosité Brookfield dudit polymère mesurée à 20°C étant comprise entre 200.000 et 800.000 mPa.s.

2. Utilisation selon la revendication 1, caractérisée en ce que le polymère de type polyacrylate a une viscosité Brookfield comprise entre 400.000 et 700.000 mPa.s.

3. Utilisation selon la revendication 1 ou 2, caractérisée en ce que R₂ est un reste de glycérol, de propylèneglycol, de butylèneglycol, d'hexylèneglycol ou un reste d'esters de glycol.

4. Utilisation selon la revendication 3, caractérisée en ce que R₁ est un groupe méthyle et R₂ est un reste de glycérol, ledit agent stabilisant ayant une viscosité Brookfield mesurée à 20°C d'environ 500.000 mPa.s.

5. Dispersion d'une phase insoluble dans l'eau dans une phase aqueuse, caractérisée en ce qu'elle contient 1 à 40 % en poids par rapport à la composition totale d'un polymère de type polyacrylate tel que défini dans l'une quelconque des revendications 1 à 4.

6. Dispersion selon la revendication 5, caractérisée en ce qu'elle contient en outre un agent gélifiant.

7. Dispersion selon l'une des revendications 5 ou 6, caractérisée en ce qu'elle contient en outre des liposomes.

8. Dispersion selon l'une quelconque des revendications 5 à 7, caractérisée en ce qu'elle est une composition cosmétique, pharmaceutique ou alimentaire.

9. Procédé pour l'obtention d'une dispersion constituée d'une phase insoluble dans l'eau dans une phase aqueuse selon l'une des revendications 5 à 8, caractérisé en ce qu'il consiste à diviser, à une température comprise entre 15 et 90°C, la phase insoluble dans l'eau en phase dispersée, dans une solution aqueuse du polymère de type polyacrylate utilisé en tant qu'agent stabilisant et si nécessaire à mélanger la phase résultante avec le complément de la phase aqueuse entre 15 et 50°C.

## Claims

1. A use as a stabilizing agent of dispersions constituted by a water-insoluble phase in an aqueous phase, of a polyacrylate-type polymer constituted by units of formula: 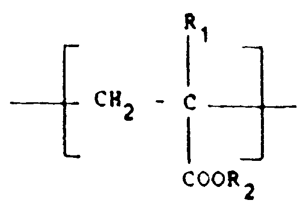 wherein :
R₂ is hydrogen or an alkyl radical having from 1 to 10 carbon atoms;
R₂ is a linear or branched hydrocarbon residue, containing from 1 to 12 carbon atoms, substituted by one or several possibly esterified hydroxy groups; the Brookfield viscosity of the said polymer measured at 20°C being comprised between 200 000 and 800 000 mPa.s.

2. A use according to claim 1, characterized in that the polyacrylate-type polymer has a Brookfield viscosity comprised between 400 000 and 700 000 mPa.s.

3. A use according to claim 1 or 2, characterized in that R₂ is a glycerol, propylene glycol, butylene glycol, hexylene glycol residue or a residue of glycol esters.

4. A use according to claim 3, characterized in that R₁ is a methyl group and R₂ is a glycerol residue, said stabilizing agent having a Brookfield viscosity measured at 20°C of approximately 500 000 mPa.s.

5. A dispersion of water-insoluble phase in an aqueous phase, characterized in that it contains from 1 to 40 % by weight in relation to the total composition of a polyacrylate-type polymer such as defined in any one of the claims 1 to 4.

6. A dispersion according to claim 5, characterized in that it further contains a gelling agent.

7. A dispersion according to one of the claims 5 or 6, characterized in that it further contains liposomes.

8. A dispersion according to any one of claims 5 to 7, characterized in that it is a cosmetic, pharmaceutical or food composition.

9. A process for obtaining a dispersion constituted by a water-insoluble phase, according to one of the claims 5 to 8, characterized in that it consists in dividing, at a temperature comprised between 15 and 90°C, the water-insoluble phase in a dispersed phase, in an aqueous solution of the polyacrylate-type polymer used as a stabilizing agent and if necessary, in mixing the resulting phase with the complement of the aqueous phase between 15 and 50°C.

## Patentansprüche

1. Verwendung eines Polymers von Type Polyacrylat als Stabilisierungsmittel für Dispersionen aus einer wasserunlöslichen Phase in einer wässrigen Phase, gemäß folgender Formel : 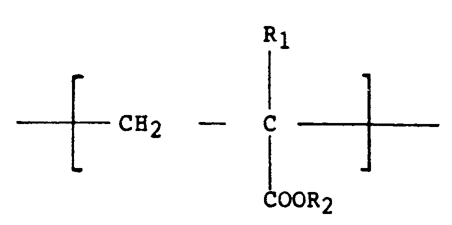 wobei R₁ Wasserstoff oder ein Alkylradikal mit 1-10 Kohlenstoffatomen ist, wobei R₂ ein Kohlenwasserstoffrest ist, geradlinig oder verzweigt, mit 1-12 Kohlenstoffatomen, substituiert durch ein oder mehrere, insbesondere veresterte Hydroxygruppen, wobei die Brookfield Viskosität des Polymers bei 20°C, zwischen 200.000 und 800.000 mPas liegt.

2. Verwendung nach Anspruch 1, dadurch gekennzeichnet, daß das Polymer von Type Polyacrylat eine Brookfield Viskosität zwischen 400.000 bis 700.000 mPas aufweist.

3. Verwendung nach Anspruch 1 oder 2, dadurch gekennzeichnet, daß R₂ ein Rest von Glycerin, Propylenglycol, Butylenglycol, Hexylenglycol oder eines Glycolesters ist.

4. Verwendung nach Anspruch 3, dadurch gekennzeichnet, daß R₁ eine Methylgruppe ist und R₂ ein Glycerinrest, wobei dieses Stabilisierungsmittel eine Brookfield Viskosität bei 20°C von ungefähr 500.000 mPas hat.

5. Dispersion einer wasserunlöslichen Phase in einer wässrigen Phase, dadurch gekennzeichnet, daß sie 1-40 Gew. % Polymer von Type Polyacrylat, gemäß irgendeinem der Ansprüche 1 bis 4 im Verhältnis zur Gesamtzusammensetzung enthält.

6. Dispersion nach Anspruch 5, dadurch gekennzeichnet, daß sie außerdem ein Geliermittel enthält.

7. Dispersion nach einem der Ansprüche 5 oder 6, dadurch gekennzeichnet, daß sie außerdem Liposome enthält.

8. Dispersion nach einem der Ansprüche 5 bis 7, dadurch gekennzeichnet, daß sie eine kosmetische, pharmazeutische oder eine Lebensmittelzusammensetzung ist.

9. Verfahren zur Herstellung einer Dispersion einer wasserunlöslichen Phase in eine wässrige Phase nach einem der Ansprüche 5 bis 8, dadurch gekennzeichnet, daß es darin besteht, bei einer Temperatur zwischen 15°C und 90°C die wasserunlösliche Phase in einer wässrigen Lösung von Polymer von Type Polyacrylat, das als Stabilisierungsmittel gebraucht wird, als dispergierte Phase zu verteilen, und wenn notwendig, die sich ergebende Phase mit der Ergänzung der wässrigen Phase zwischen 15°C und 50°C zu mischen.
